# EUROPEAN PATENT APPLICATION

(11) **EP 4 245 259 A1**
(43) Date of publication of application: **20.09.2023**
(21) Application number: 21905490.5
(22) Date of filing: 26.11.2021
(51) Int. Cl.: A61F 2/24

(54) **MITRAL VALVE CLIP HAVING LOCKING MECHANISM**

(30) Priority: 18.12.2020 CN 202011511245
(71) Applicant: Jenscare Scientific Co., Ltd., Ningbo, Zhejiang 315336 (CN)
(72) Inventor: LV, Shiwen, Hangzhou Bay New District Ningbo, Zhejiang 315336 (CN); CHEN, Zhi, Hangzhou Bay New District Ningbo, Zhejiang 315336 (CN); LU, Kan, Hangzhou Bay New District Ningbo, Zhejiang 315336 (CN); WU, Lei, Hangzhou Bay New District Ningbo, Zhejiang 315336 (CN)
(74) Representative: Appleyard Lees IP LLP
(86) International application number: PCT/CN2021/133489
(87) International publication number: WO 2022/127561

(57) **Abstract**

A mitral valve clip having a locking mechanism. The mitral valve clip comprises a first clamping arm (1), a second clamping arm (2), a linking member (3), a push-pull device (9), and a locking device (4). The locking device (4) matches the linking member (3), and the locking device (4) comprises a locking head (41) and a self-locking rod (42); the linking member (3) is respectively hinged to the first clamping arm (1) and the second clamping arm (2), and the first and second clamping arms (1, 2) respectively comprise a long arm (12) and a short arm (11); one end of the long arm (12) is connected to the push-pull device (9) in a hinge manner, and one end of the short arm (11) is provided with a locking portion (111). When the push-pull device (9) is operated to enable an opening angle of the long arm (12) of the first clamping arm (1) and the long arm (12) of the second clamping arm (2) to become larger and be in an opening state, the locking portion (111) on the first clamping arm (1) and the locking portion (111) on the second clamping arm (2) are in a staggered matching state. When the push-pull device (9) is operated to enable an opening angle of the long arm (12) of the first clamping arm (1) and the long arm (12) of the second clamping arm (2) to become smaller and be in a closing state, the locking device (4) is moved to enable the locking head (41) and the locking portion (111) to achieve matching and locking.

## Description

This application claims to the priority of Chinese Patent Application No. 202011511245.2, filed on December 18, 2020, entitled "VALVE CLIP WITH LOCKING MECHANISM", the entire contents of which is incorporated herein by reference.

### TECHNICAL FIELD

The present application relates to the field of medical devices, and in particular, to a valve clip with a locking mechanism.

### BACKGROUND

The anatomical structure of the mitral valve is complex, including leaflets, valve ring, chordae, and papillary muscles, which play an important role in maintaining the function of the left and right ventricles respectively. Any disease that affects the structural integrity and normal function of the leaflets, valve ring, chordae, papillary muscle, and left ventricle may lead to severe mitral regurgitation (MR), which can cause left ventricular failure, pulmonary hypertension, atrial fibrillation, stroke and death. According to the latest epidemiological survey data of western developed countries such as the United States, mitral valve regurgitation is the most common type of valvular disease in the elderly population over 65 years old. At present, although there is no authoritative epidemiological survey data in my country, with the advent of population aging, there is no doubt that the number of patients with mitral valve regurgitation in my country is huge. The mitral valve insufficiency can be divided into degenerative MR and functional MR. The degenerative MR is caused by pathological changes in one or more of the leaflets, valve ring, chordae, and papillary muscles. The functional MR usually refers to left ventricular function abnormalities, such as enlarged valve ring, but the mitral valve is usually normal.

At present, the treatment methods of MR mainly include drug therapy, surgery, and interventional therapy. The drug therapy can only improve the patient's symptoms, but cannot prolong the patient's survival time. The surgery, which mainly refers to valve repair or valve replacement, is recognized as the preferred treatment of choice for mitral regurgitation, and has been proven to relieve symptoms and prolong life of patients. However, for many elderly high-risk patients with multi-system diseases, the surgical risk is high and the survival benefit is small. According to European data, the surgical success rate of such patients is only 50%, and the surgical success rate of patients with severe functional MR is even as low as 16%. Therefore, transcatheter mitral valve repair and replacement can theoretically benefit high-risk patients who have lost the opportunity for surgery. Intervention therapy is to load an artificial implant on a delivery system in vitro, deliver it to the mitral valve ring, along the vascular path or by puncturing the cardiac apex, and then release and fix it to completely or partially replace the function of the native valve. At present, the mitral valve interventional therapy has become one of the research hotspots in related fields, and many products are under development. However, due to the complexity of the mitral valve itself and its surrounding structures and etc., the development of mitral valve interventional devices faces many special difficulties.

Patent CN103826548A provides a method for fixing tissue, the method includes: providing an implantable fixation device, which includes a pair of fixation elements, each fixation element has first ends, free ends opposite to the first ends, and engaging surfaces located between the first ends and the free ends to engage the tissue. The first ends are movably coupled together such that the fixation element can move between a closed position in which the engaging surfaces faces each other and a first open position in which the engaging surfaces are positioned away from each other. A locking mechanism in this patent includes one or more wedge-shaped elements, such as rolling elements. The rolling elements includes a pair of barbells arranged on opposite sides of as stud. The barbell is operated by a hooked end of a releasing appliance. When upward force is applied to the appliance through a locking wire, the barbell is lifted by the hooked end against a spring, such that the barbell is pulled upwards along a sidewall or an inclined surface, and is disengaged from the stud. The upward force on the barbell is released by the hooked end, such that the spring forces the barbell downward, and the barbell is wedged between a surface of the apparatus and the stud, which limits the movement of the stud, which in turn locks a detent mechanism and a distal element in place. The locking mechanism of the invented MitraClip has technical defects lying in that: the design of the locking structure is relatively complicated, and the locking needs to be completed through the cooperation between the rolling elements, the springs, the locking wires, the surface of the wedge-shaped apparatus and the stud, which requires high assembly technology. The spring is in a force-deformed state in an initial state, and returns to its original shape after the locking wire is released, so the locking structure has no reversibility. If an operation error occurs during the operation and the valve having not been clamped, the locking mechanism cannot be unlocked, which means the operation being failed. In this case, the implanted device remains in the body after the operation, and the spring will be fatigued after being stressed for a long time, which will affect the clipping force of the leaflets, so the unlocking mechanism has potential safety hazards.

The Chinese Patent application No. 201880066104.9 discloses a valveable clip device, including a spacer member configured to be arranged between leaflets of a natural heart valve located between a first chamber and a second chamber of the heart. A prosthetic device further includes a plurality of anchoring members. The plurality of anchoring members are coupled to the spacer member, and configured to trap the leaflets between the respective anchoring members and the spacer member such that the prosthetic device are held between the leaflets. When the leaflets are trapped between the anchoring member and the spacer member, the spacer member is configured to provide a flow path through the prosthetic device between the first chamber and the second chamber, such that blood can pass through the spacer member, and countercurrently flows from the second chamber to the first chamber. The technical solution of this patent uses pre-shaped metal material to clamp and secure the original leaflets. Although a transmission system thereof is simplified, since there is no reliable locking device and the heart is constantly contracting and relaxing, the original leaflets are easy to slip off from between the spacer member and the anchoring member. Moreover, using the pre-shaped metal material to clamp and secure the original leaflets is prone to fatigue damage, which affects its service life.

As described above, due to the relatively complex design of the locking structure in the prior art, the requirements for the assembly process are extremely high, and the spring sheet become fatigued after a long time, which will affect the clipping force. Therefore, there is a need for a locking mechanism that is relatively simple in structural design, easy to assemble, and can maintain the clipping force for a long time.

### SUMMARY

An object of the present application is to provide a valve clip with a locking mechanism, which has the advantages of being simple in structure, low in requirement for an assembly process, and capable of maintaining a clipping force for a long time.

In order to solve the above technical problems, the present application provides the following technical solutions. A valve clip with a locking mechanism includes a first clipping arm, a second clipping arm, a connecting member, a push-pull device, and a locking device. The locking device partially cooperates with the connecting member. The locking device includes a locking head and a self-locking rod. The connecting member is hinged to the first clipping arm and the second clipping arm respectively. The clipping arm includes a long arm and a short arm. An end of the long arm is hinged to the push-pull device, and an end of the short arm is provided with a locking part. When the push-pull device is operated such that an angle formed between the long arm of the first clipping arm and the long arm of the second clipping arm becomes large and is in an open state, the locking part of the first clipping arm and the locking part of the second clipping arm are partially in a staggered fitting state. When the push-pull device is operated such that the angle formed between the long arm of the first clipping arm and the long arm of the second clipping arm becomes small and is in a closed state, the locking device is moved such that the locking head cooperates with and locks the locking part.

The present application can also be further implemented through the following technical solutions.

In an embodiment, a locking area is formed between the locking part and the connecting member. A size of the locking area changes with the angle formed between the clipping arms. When the angle formed between the first clipping arm and the second clipping arm becomes small, the locking area gradually becomes large. When the locking head enters the locking area and completes locking cooperation with the locking part, the valve clip is locked.

In an embodiment, an offset structure is arranged on the long arm. The long arm is divided into a fitting portion and a transmission portion by the offset structure. When the angle formed between the long arm of the first clipping arm and the long arm of the second clipping arm becomes small and is in the closed state, the fitting portion is arranged at a position closer to a center axis of the valve clip than the transmission portion.

In an embodiment, a rotating structure is arranged between the self-locking rod and the locking head, so that the self-locking rod and the locking head is capable of rotating relative to each other.

In an embodiment, the connecting member includes a connecting block and connecting lugs arranged on the connecting block. The connecting block is connected to the locking device. The connecting lugs are hinged connected to the first clipping arm and the second clipping arm respectively.

In a preferred embodiment, the connecting member is in an arc shape or in a "V" shape. The purpose of this design is to shorten a loading length of the valve clip when pre-installed.

In a preferred embodiment, the connecting lugs are axially symmetrical about a central axis of the connecting block.

In a preferred embodiment, the connecting block is provided with a mounting groove, at least part of the locking head is always arranged in the mounting groove, and the mounting groove restricts the circumferential rotation of the locking head. The purpose of this design is that: when the locking head is arranged in a special-shaped structure, the self-locking rod is operated such that during the locking head axially moving to cooperate with and lock the locking part, the mounting groove restricts the circumferential rotation of the locking head, so that the locking head enters the locking area according to a predetermined position and completes cooperation with the locking part.

In a preferred embodiment, a through hole is arranged on the connecting block in an axial direction. The mounting groove is arranged on a distal side of the through hole.

In an embodiment, a movement of the locking head towards the proximal end is restricted by the mounting groove, and a diameter of the mounting groove is greater than a diameter of the through hole.

In an embodiment, the self-locking rod is solid or hollow.

In a preferred embodiment, when the locking area formed by the locking part is a wedge-shaped structure or the locking area is a tapered structure, the locking head is correspondingly arranged to be a wedge-shaped structure or tapered structure. When both locking area and locking head are a wedge-shaped or tapered structure, the locking force is stronger and the valve clip is more stable and reliable.

In an embodiment, the locking head is a stent-shaped self-expanding structure. The locking head cooperates with the locking part to realize locking.

In an embodiment, the locking head is in a tapered structure, a shuttle-shaped structure, a diamond-shaped structure, a prismatic structure, or an arrowhead-shaped structure.

In a preferred embodiment, the locking head is hollow. The locking head is made of an elastic metal material. A hollow area is formed inside the locking head, which is a buffering area. When the valve clip is implanted in the heart, the clipping arms will be subject to certain stress due to the compression and relaxation of the heart valve itself, which will affect the clipping degree between the clipping arms and the pushing device, and a certain degree of reflux may occur. However, when the locking head is made of the elastic metal material, and the buffering area is formed inside the locking head, the stress received by the long arm of the clipping arm due to the valve's own movement is transmitted to the short arm, and then the stress received by the short arm is transmitted to the locking head. The locking head is deformed to a certain extent, so that the stress received by the locking head is decomposed, and the clipping strength and tightness between the clipping arm and the push-pull device are always ensured, thereby avoiding reflux.

In an embodiment, the short arm is in an arc shape or "L" shape. When the angle formed between the first clipping arm and the second clipping arm becomes large, the locking part of the first clipping arm and the locking part of the second clipping arm are staggered, and fitted into each other. The locking part is designed as the arc-shaped segment or to be in a "L" shape, helping increase the force arm of the locking part, and enhance the locking force. In addition, the staggered fitting method is beneficial to save the loading space.

In an embodiment, the connecting member is provided with an anti-retraction device cooperating with the self-locking rod. The anti-retraction device can enhance the stability of the locking device and ensure the locking effect for a long time.

In a preferred embodiment, the anti-retraction device is a thread or a groove.

In an embodiment, the push-pull device includes leaflet capturing devices, a leak-proof tube, a first linkage rod, and a second linkage rod, and the first linkage rod and the second linkage rod are hinged to the leak-proof tube and arranged on left and right sides of the leak-proof tube. The first linkage rod and the second linkage rod are hinged to the first clipping arm and the second clipping arm respectively. The leaflet capturing devices are arranged on the first linkage rod and the second linkage rod. The leaflet capturing device has a preset shape. In the natural state, the leaflet capturing devices are tightly attached to the linkage rods. During pre-installation, the leaflet capturing device is always attached to the leak-proof tube. When the valve clip captures the leaflet, the leaflet capturing device returns to the preset shape to clamp the leaflet.

In a preferred embodiment, the leaflet capturing device can be controlled separately, to respectively clamp and anchor individual leaflets, which helps reduce the difficulty of operation.

In an embodiment, the locking part includes a plurality of arc-shaped rods distributed in a comb shape. When the angle formed between the first clipping arm and the second clipping arm becomes large, the arc-shaped rod of the first clipping arm and the arc-shaped rod of the second clipping arm are staggered and fitted into each other. The plurality of arc-shaped rods distributed in a comb shape help to increase the number of action points of the force on the short arm, which makes the locking more stable and reliable.

In a preferred embodiment, an end of the arc-shaped rod is bent inward, and the arc-shaped rod provides a point of force to the locking device when the first clipping arm and the second clipping arm are in a closed state.

In an embodiment, a cross-section of the long arm is in a concave shape, the purpose of this design is that: when the valve clip is in a closed state, the long arm plays a certain role in wrapping the leak-proof tube, so that the leak-proof tube has a better leak-proof effect. In addition, the arrangement of the concave shape also greatly reduces the weight of the long arm, effectively preventing the valve clip from slipping off in the heart due to excessive weight, which is beneficial to the stability of the valve clip anchored in the heart, and the original leaflet is not excessively torn, and the heart tissue is not damaged.

In an embodiment, an end of the long arm connected to the push-pull device is provided with an arc buffering section. This design has advantages lying in that when the valve clip captures the leaflet and completes the clipping, the arc buffering section can protect the original leaflet from being damaged.

In an embodiment, the clipping arm is provided with a fitting structure at the hinge point. Advantages of arranging the fitting structure lie in that the short arm levers the long arm with the hinge point, such that when locking, the force at the hinge point is relatively large, and the fitting structure can increase the contact surface with the connecting member, so that the locking moment is more balanced.

Compared with the prior art, the present application has the advantages as follows.
1. Different from the prior art, in the present application, when the push-pull device is operated such that the angle formed between the long arm of the first clipping arm and the long arm of the second clipping arm becomes large and in an open state, the locking part of the first clipping arm and the locking part of the second clipping arm are in a staggered fitting state, which will not interfere/affect the magnitude of the angle formed between the clipping arms, which is beneficial to capture and clamp the leaflets. When the valve clip is required to be locked, the locking device is moved so that the cooperation and locking between the locking head and the locking part can be realized, that is, the locking is completed. The principle of leverage is utilized, so that a torque balance is generated between an end of the locking device and the locking part to control the angle formed between the first clipping arm and the second clipping arm to be no longer increased to achieve the locking effect. In addition, the locking structure achieves a mechanical locking, which is simple in structure, easy to operate and has no risk of fatigue, and can guarantee the clipping effect for a long time.
2. Different from the prior art, the short arm of the present application is arc-shaped or L-shaped. The purpose of this design is that the arc-shaped section can increase the force arm of the locking part, thereby ensuring the locking force of the valve clip, so that the locking state of the valve clip is more stable and reliable, and the staggered fitting method is beneficial to save the loading space.
3. Different from the prior art, in the present application, the connecting member is provided with a mounting groove, at least part of the locking head is always arranged in the mounting groove, and the mounting groove restricts the circumferential rotation of the locking head. The purpose of this design is that: when the locking head is arranged in a special-shaped structure, the self-locking rod is operated such that during the locking head axially moving to cooperate with and lock the locking part, the mounting groove restricts the circumferential rotation of the locking head, so that the locking head enters the locking area according to a predetermined position and completes cooperation with the locking part. When the mounting groove is arranged on the connecting member, the weight of the valve clip can be reduced, which can effectively prevent the valve clip from slipping off in the heart due to excessive weight, and is beneficial to the stability of the valve clip anchored in the heart.
4. Different from the prior art, the cross-section of the long arm is in a concave shape. The purpose of this design is that: when the valve clip is in a closed state, the long arm plays a certain role in wrapping the leak-proof tube, so that the leak-proof tube has a better leak-proof effect. In addition, the arrangement of the concave shape also greatly reduces the weight of the long arm, effectively preventing the valve clip from slipping off in the heart due to excessive weight, which is beneficial to the stability of the valve clip anchored in the heart, and the original leaflets is not excessively torn, and the heart tissue is not damaged.
5. Different from the prior art, the locking head of the present application is a stent-shaped self-expanding structure, and a hollow area is formed inside the locking head, which is a buffering area. When the valve clip is implanted in the heart, the clipping arms will be subject to certain stress due to the compression and relaxation of the heart valve itself, which will affect the clipping degree between the clipping arms and the pushing device, and a certain degree of reflux may occur. However, when the locking head is made of the elastic metal material, and the buffering area is formed inside the locking head, the stress received by the long arm of the clipping arm due to the valve's own movement is transmitted to the short arm, and then the stress received by the short arm is transmitted to the locking head. The locking head is deformed to a certain extent, so that the stress received by the locking head is decomposed, and the clipping strength and tightness between the clipping arm and the push-pull device are always ensured, thereby avoiding reflux.
6. Different from the prior art, in the present application, the offset structure is provided. The offset structure enables the fitting portion to be arranged at a position closer to the center axis of the valve clip than the transmission portion when the angle formed between the long arm of the first clipping arm and the long arm of the second clipping arm becomes small and is in the closed state, so that when the valve clip is in a closed state, the long arm can be tightly attached to the leak-proof tube, which further ensures the clipping force of the valve clip and also improves the leak-proof effect.

### BRIEF DESCRIPTION OF THE DRAWINGS

FIGS. 1a to 1d are schematic views showing a locking process realized by a valve clip according to the present application.
FIGS. 2a to 2h are schematic views showing clipping arms, locking parts, arc-shaped rods according to the present application.
FIGS. 3a to 3g are schematic views showing a locking device and a locking block according to the present application, and FIGS. 3c to 3g illustrates some embodiments of the locking block.
FIGS. 4a to 4d are schematic views showing a connecting member according to the present application.
FIGS. 5a to 5b are schematic views of a disassembling process of a delivering catheter and a valve clip according to the present application.
FIGS. 6a to 6g are schematic views of a process of the delivering catheter entering the heart of the present application.
FIGS. 7a to 7c are schematic views of a process of opening the valve clip in the left atrium according to the present application.
FIGS. 8a to 8h are schematic views of a process of the valve clip moving to the optimal position for capturing the valve according to the present application.
FIGS. 9a to 9c show a locking device for locking the valve clip according to another embodiment of the present application.
FIGS. 10a to 10c show a locking device for locking the valve clip according to another embodiment of the present application.
FIGS. 11a to 11g show a locking device for locking the valve clip according to another embodiment of the present application.

### Illustration for reference signs:

1, first clipping arm; 11, short arm; 12, long arm; 13, fitting structure; 111, locking part; 121, arc buffering section; 122, offset structure; 123, fitting portion; 124, transmission portion; 1111, arc-shaped segment; 1112, first arc-shaped rod; 2, second clipping arm; 3, connecting member; 31, connecting block; 311, through hole; 312, mounting groove; 32, connecting lug; 33, locking area; 34, anti-retraction device; 4, locking device; 41, locking head; 42, self-locking rod; 43, rotating structure; 431, groove; 432, protruding portion; 5, leak-proof tube; 51, leak-proof tube connecting portion; 6, first linkage rod; 7, second linkage rod; 8, delivery catheter; 9, push-pull device; 91, leaflet capturing device.

### DETAILED DESCRIPTION OF THE EMBODIMENTS

The present application will be further described in detail below in conjunction with the accompanying drawings and examples.

A proximal end in the present application refers to an end adjacent to an operator, and a distal end refers to an end away from the operator.

### First specific Example

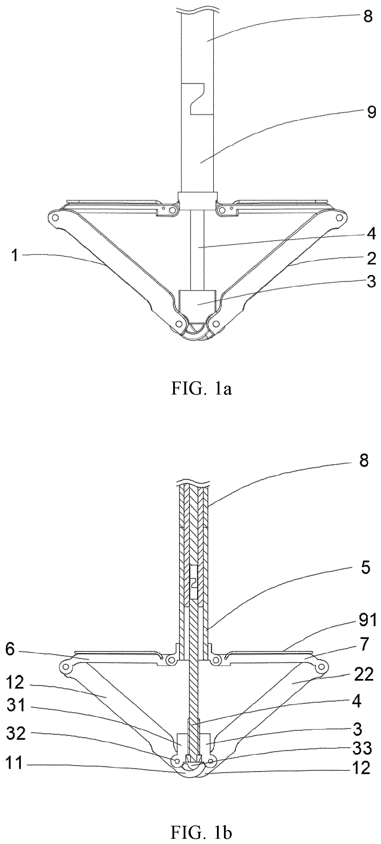

When used for the treatment of mitral valve diseases, as shown in FIGS. 1a to 1d, a valve clip with a locking mechanism includes a first clipping arm 1, a second clipping arm 2, a connecting member 3, a push-pull device 9, and a locking device 4. The locking device 4 partially cooperates with the connecting member 3. The locking device 4 includes a locking head 41 and a self-locking rod 42. The connecting member 3 is hinged to the first clipping arm 1 and the second clipping arm 2 respectively. The clipping arm includes a long arm 12 and a short arm 11. An end of the long arm 12 is hinged to the push-pull device 9, and an end of the short arm 11 is provided with a locking part 111. When the push-pull device 9 is operated such that an angle formed between the long arm 12 of the first clipping arm 1 and the long arm 12 of the second clipping arm 2 becomes large and is in an open state, the locking part 111 of the first clipping arm 1 and the locking part 111 of the second clipping arm 2 are partially in a staggered fitting state. When the push-pull device 9 is operated such that the angle formed between the long arm 12 of the first clipping arm 1 and the long arm 12 of the second clipping arm 2 becomes small and is in a closed state, the locking device 4 is moved such that the locking head 41 cooperates with and locks the locking part 111.

The assembly and connection mode of various components of the valve clip with the locking mechanism of the present application will be described in detail below in conjunction with the accompanying drawings.

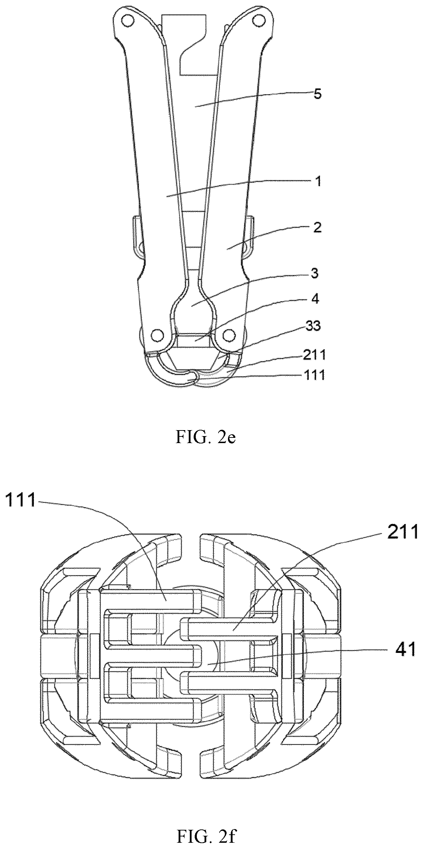

In this example, a locking area 33 is formed between the locking part 111 and the connecting member 3. A size of the locking area 33 changes with the angle formed between the clipping arms. When the angle formed between the first clipping arm 1 and the second clipping arm 2 becomes small, the locking area 33 gradually becomes large. When the locking head 41 enters the locking area 33 and completes locking cooperation with the locking part 111, the valve clip is locked, as shown in FIGS. 2e and 2f.

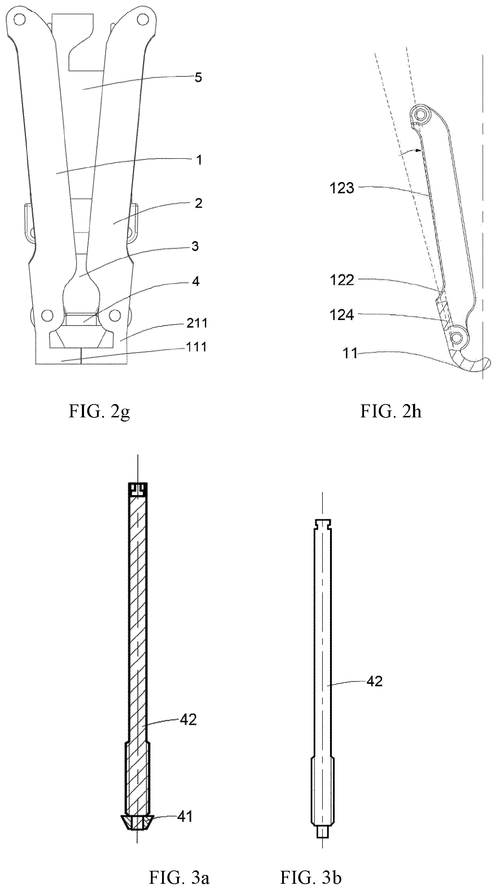

In this example, an offset structure 122 is arranged on the long arm 12, as shown in FIG. 2h. The long arm 12 is divided into a fitting portion 123 and a transmission portion 124 by the offset structure 122. When the angle formed between the long arm 12 of the first clipping arm 1 and the long arm 12 of the second clipping arm 2 becomes small and is in the closed state, the fitting portion 123 is arranged at a position closer to a center axis of the valve clip than the transmission portion 124. Advantages of this design is that when the valve clip is in the closed state, the long arm 12 can be closely attached to the push-pull device 9, further increasing the clipping effect, and improving the leak-proof function.

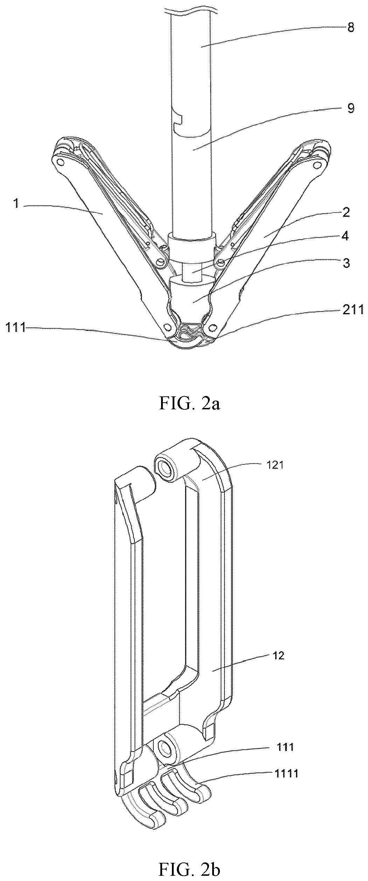

In this example, the locking part 111 includes an arc-shaped segment 1111, as shown in FIGS. 2a and 2b. When the angle formed between the first clipping arm 1 and the second clipping arm 2 becomes large, the arc-shaped segment 1111 of the first clipping arm 1 and the arc-shaped segment 2111 of the second clipping arm 2 are staggered, and fitted into each other. The locking part 111 is designed as the arc-shaped segment 1111 to help increase the force arm of the locking part 111, and enhance the locking force. In addition, the staggered fitting method is beneficial to save the loading space. Similarly, the locking part 111 can also be arranged in an "L" shape, as shown in FIG. 2g.

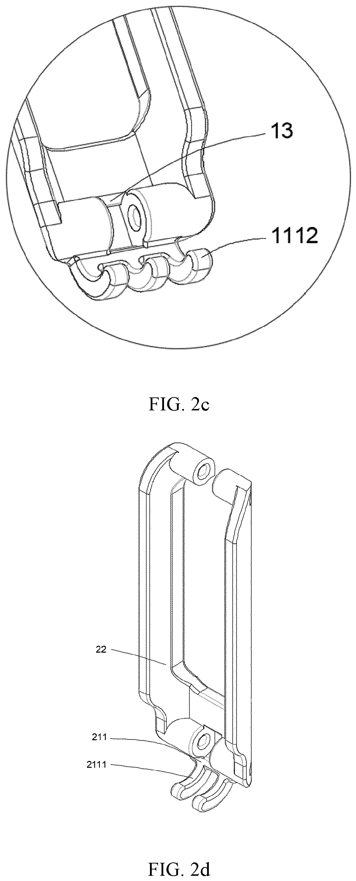

In this example, the locking part 111 includes a plurality of arc-shaped rods 1112, as shown in FIG. 2c. When the angle formed between the first clipping arm 1 and the second clipping arm 2 becomes large, the arc-shaped rod 1112 of the first clipping arm 1 and the arc-shaped rod 1112 of the second clipping arm 2 are staggered and fitted into each other. The plurality of arc-shaped rods 1112 help to increase the number of action points of the force on the short arm 11, which makes the locking more stable and reliable.

In this example, the first clipping arm 1 has three arc-shaped rods 1112, and the second clipping arm 2 has two arc-shaped rods 2112, as shown in FIGS. 2b and 2d. On the premise of ensuring the strength of the arc-shaped rod 1112, an end of the arc-shaped rod 1112 touches the locking head 41.

In this example, the clipping arm is provided with a fitting structure 13 at the hinge point, as shown in FIG. 2c. Advantages of arranging the fitting structure 13 lie in that the short arm 11 levers the long arm with the hinge point, such that when locking, the force at the hinge point is relatively large, and the fitting structure 13 can increase the contact surface with the connecting member 3, so that the locking moment is more balanced.

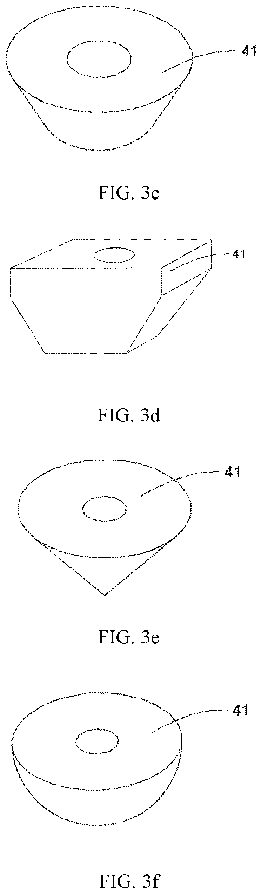

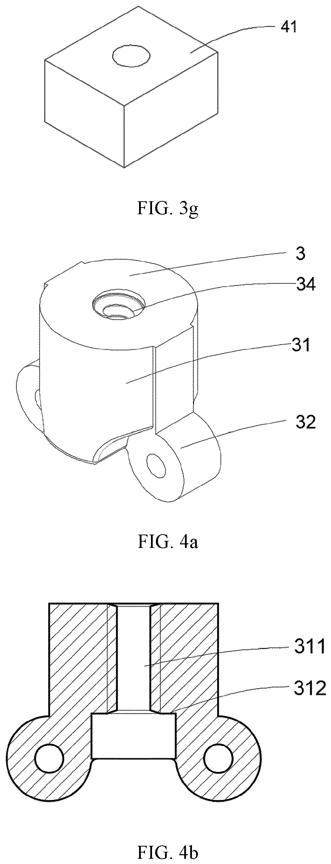

In this example, the locking device 4 includes a locking head 41 and a self-locking rod 42, as shown in FIGS. 3a and 3b. The locking head 41 and the locking part 111 on the short arm 11 are in a trapezoidal cooperation, a wedge-shaped cooperation, a tapered cooperation, a round face cooperation, or a cube cooperation, as shown in FIGS. 3c-3g.

In this example, the connecting member 3 includes a connecting block 31 and connecting lugs 32 arranged on the connecting block 31, as shown in FIGS. 4a and 4b. The connecting block 31 is connected to the locking device 4. The connection ears 32 are hinged to the first clipping arm 1 and the second clipping arm 2 respectively.

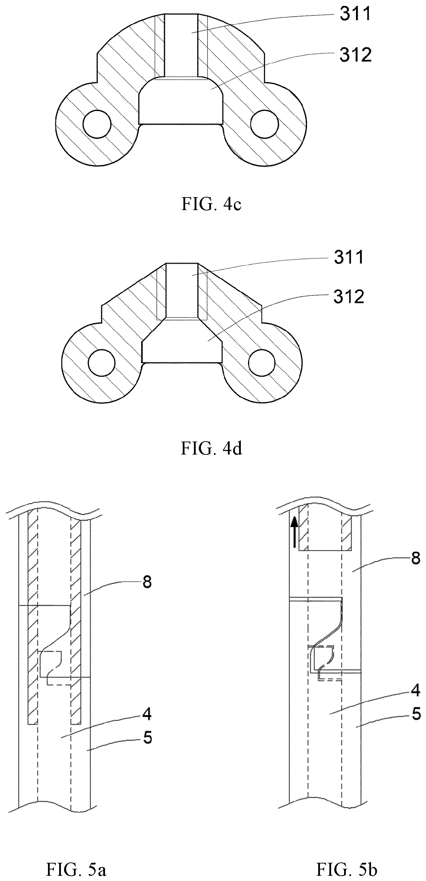

In this example, the connecting member 3 may be in an arc shape or in a "V" shape, as shown in FIGS. 4c and 4d. The purpose of this design is to shorten a loading length of the valve clip during pre-installation.

In this example, the connecting member 3 is provided with an anti-retraction device 34 cooperating with the self-locking rod 42. The anti-retraction device 34 can enhance the stability of the locking device 4 and ensure the locking effect for a long time.

In this example, the anti-retraction device 34 is a thread or a groove.

In this example, the push-pull device 9 includes leaflet capturing devices 91, a leak-proof tube 5, a first linkage rod 6, and a second linkage rod 7, and the first linkage rod 6 and the second linkage rod 7 are hinged to the leak-proof tube 5 and arranged on left and right sides of the leak-proof tube 5. The first linkage rod 6 and the second linkage rod 7 are hinged to the first clipping arm 1 and the second clipping arm 2 respectively. The leaflet capturing devices 91 are arranged on the first linkage rod 6 and the second linkage rod 7. The leaflet capturing device 91 has a preset shape. In the natural state, the leaflet capturing devices 91 are tightly attached to the linkage rods. During pre-installation, the leaflet capturing device 91 is always attached to the leak-proof tube 5. When the valve clip captures the leaflet, the leaflet capturing device 91 returns to the preset shape to clamp the leaflet.

In this example, the end of the long arm 12 connected to the push-pull device 9 is provided with an arc buffering section 121, as shown in FIGS. 2a and 2b. When the valve clip captures the leaflet and completes the clipping, the arc buffering section 121 can protect the original leaflet from being damaged.

In this example, a cross-section of the long arm 12 is in a concave shape, the purpose of this design is that: when the valve clip is in a closed state, the long arm 12 plays a certain role in wrapping the leak-proof tube 5, so that the leak-proof tube 5 has a leak-proof effect. In addition, the arrangement of the concave shape also greatly reduces the weight of the long arm 12, effectively preventing the valve clip from slipping off in the heart due to excessive weight, which is beneficial to the stability of the valve clip anchored in the heart, and the original leaflets is not excessively torn, and the heart tissue is not damaged.

In this example, the valve clip further includes a valve clip delivery system. The valve clip delivery system includes a control handle and a delivery catheter 8 connected to the control handle. As shown in FIGS. 5a-5b. A distal end portion of the delivery catheter 8 is detachably connected to the valve clip. When pre-installed, the delivery catheter 8 is connected to the valve clip. When the valve clip is implanted at the target position, the delivery catheter 8 is separable from the valve clip.

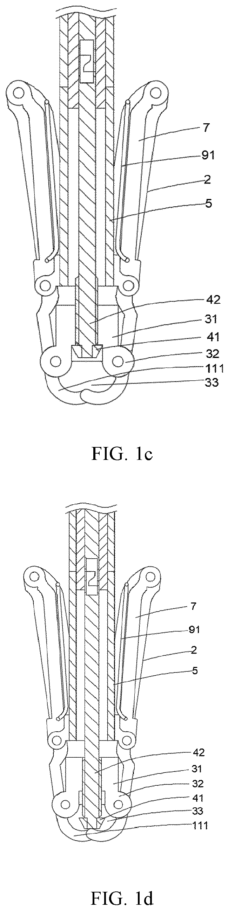

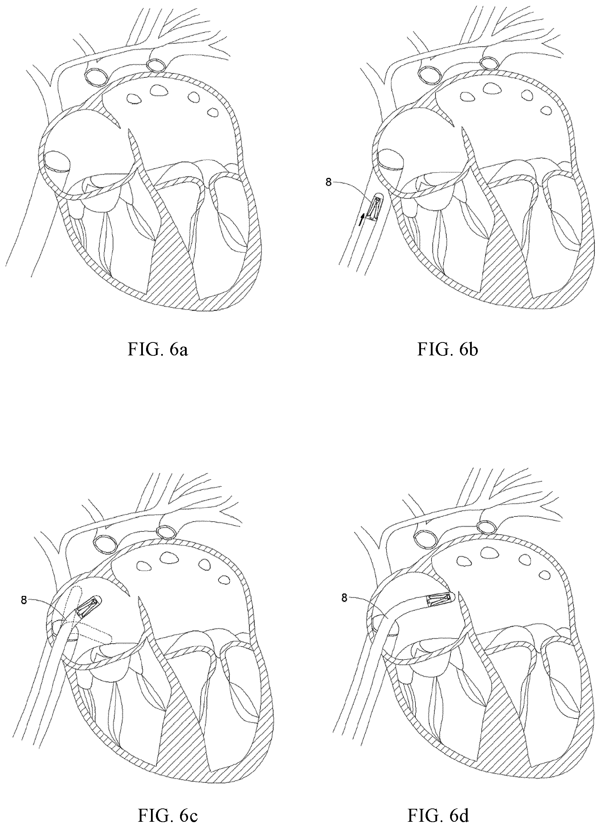

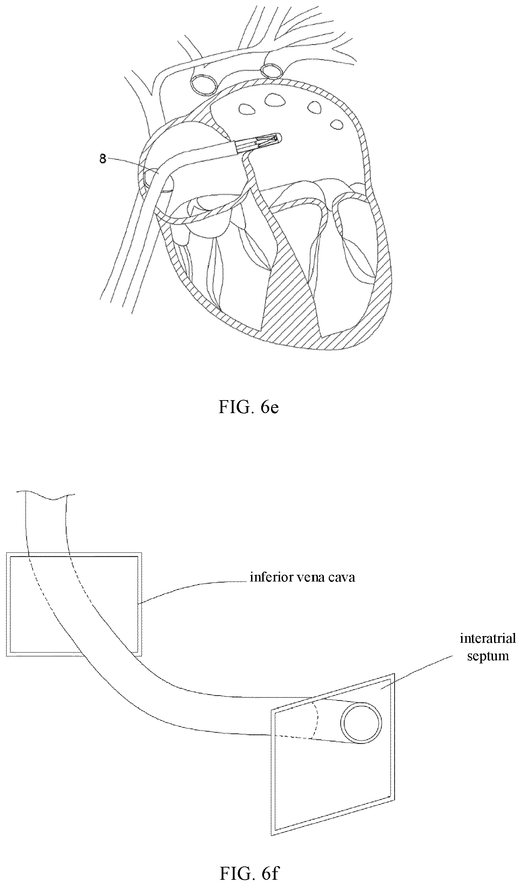

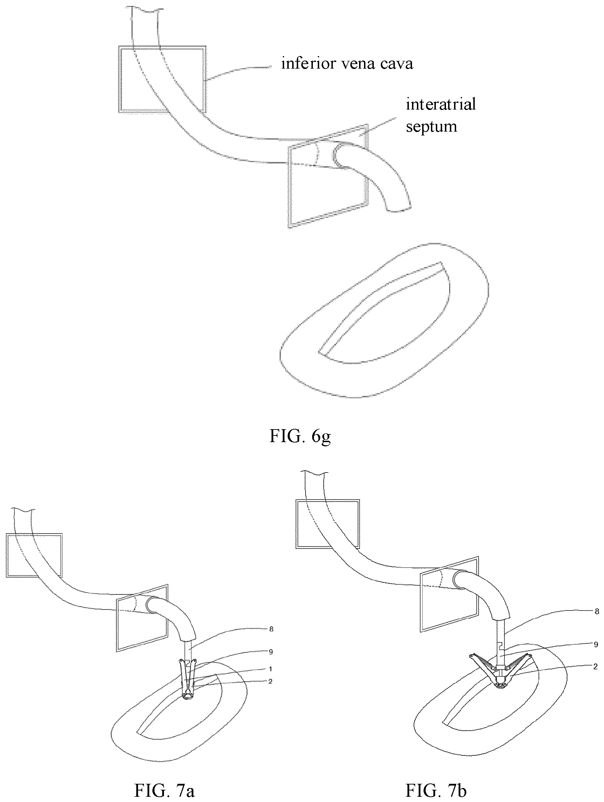

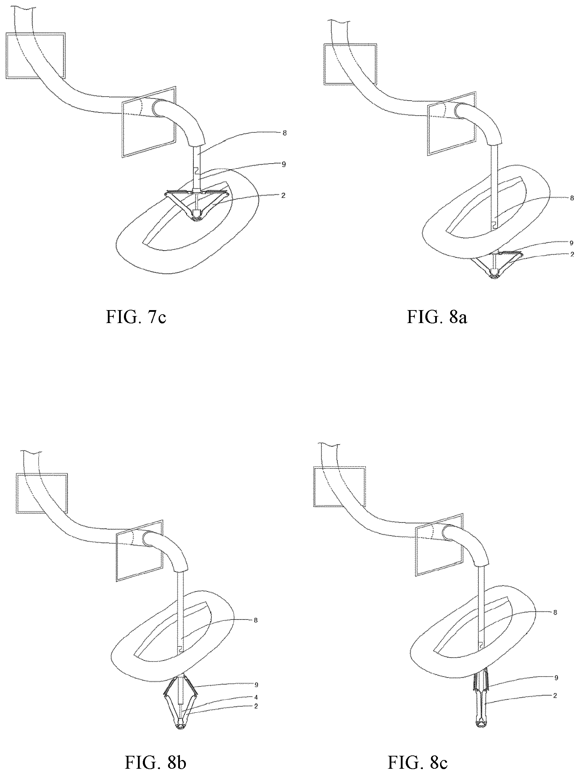

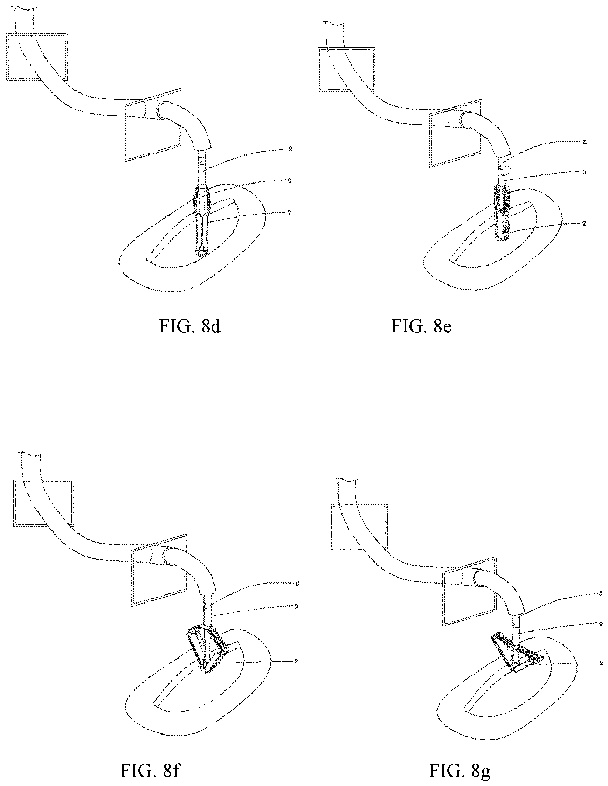

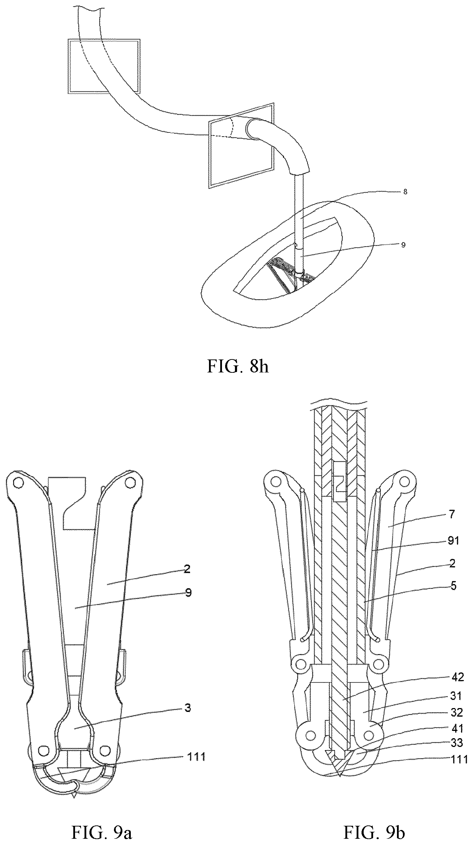

Working principle of the present application is as follows.
1. The valve clip delivery catheter 8 is operated to enter the heart from the inferior vena cava, and then, the delivery catheter 8 is operated so that the valve clip passes through the interatrial septum, as shown in FIGS. 6a to 6e. The delivery catheter 8 is continually operated to bend so that the valve clip faces the mitral valve, as shown in FIGS. 6f and 6g.
2. The control handle is operated, and the outer sheath thereof is then withdrawn to expose the valve clip in the left atrium. The control handle is operated to move the locking device 4 toward the distal end relative to the leak-proof tube 5 until the angle formed between the first clipping arm 1 and the second clipping arm 2 becomes largest, as shown in FIGS. 7a~7c.
3. The control handle is operated so that the valve clip passes through the mitral valve, as shown in FIG. 8a. It is determined whether the valve clip is in the optimal valve clipping position through an image, and if the valve clip is in the optimal position, it proceeds directly to step 5; if the valve clip is not in the optimal position, it proceeds to step 4.
4. The control handle is operated so that the locking device 4 is pulled farthest. In this case, the radial space occupied by the valve clip is the smallest, and the valve clip is pulled to the left atrium, as shown in FIGS. 8b-8d. An obtuse angle is formed between the clipping arm and the linkage rod, and is in line with the physiological structure, and will not damage the cardiac tissues such as chordae. The control handle is operated to rotate and deliver the valve clip to the optimal valve clipping position, as shown in FIGS. 8e to 8h.
5. The control handle is operated to clip the leaflets so that the front part and the rear part of the leaflets enter the leaflet capturing device 91, and are respectively fixed between the leak-proof tube 5 and the first linkage rod 6, and between the leak-proof tube fitting 5 and the second linkage rod 7.
6. The control handle is operated to move the locking device 4 toward the proximal end until the angle formed between the first clipping arm 1 and the second clipping arm becomes smallest, as shown in FIG. 1c.
7. The locking device 4 is operated to move axially toward the distal end along the connecting member 3 until the locking head 41 abuts against the first locking part 111 and the second locking part 111 to achieve the purpose of locking the valve clip, as shown in FIG. 1d.
8. The control handle is operated to separate the delivery catheter 8 from the valve clip, as shown in FIGS. 5a and 5b, which illustrates that the delivery catheter 8 is withdrawn from the body.

### Second specific Example

In another embodiment, as shown in FIGS. 9a and 9b, a valve clip with a locking mechanism includes a first clipping arm 1, a second clipping arm 2, a connecting member 3, and a locking device 4. The locking device 4 is connected to the connecting member 3. The connecting member 3 is hinged to the first clipping arm 1 and the second clipping arm 2 respectively. The clipping arm includes a long arm 12 and a short arm 11. The short arm 11 levers the long arm 12 with a hinge point. An end of the short arm 11 is provided with a locking part 111. When an angle formed between the first clipping arm 1 and the second clipping arm 2 becomes large, the locking part 111 of the first clipping arm 1 and the locking part 111 of the second clipping arm 2 are staggered, and fitted into each other. When the angle formed between the first clipping arm 1 and the second clipping arm 2 becomes small, a locking area 33 is formed between the locking part 111 and the connecting member 3, and an end of the locking device 4 enters the locking area 33 and completes locking cooperation with the locking part 111.

The valve clip further includes a push-pull device 9. The push-pull device 9 includes leaflet capturing devices 91, a leak-proof tube 5, a first linkage rod 6, and a second linkage rod 7, and the first linkage rod 6 and the second linkage rod 7 are hinged to the leak-proof tube 5 and arranged on left and right sides of the leak-proof tube 5. The first linkage rod 6 and the second linkage rod 7 are hinged to the first clipping arm 1 and the second clipping arm 2 respectively. The leaflet capturing devices 91 are arranged on the first linkage rod 6 and the second linkage rod 7. The leaflet capturing device 91 has a preset shape. In the natural state, the leaflet capturing devices 91 are tightly attached to the linkage rods. During pre-installation, the leaflet capturing device 91 is always attached to the leak-proof tube 5. When the valve clip captures the leaflet, the leaflet capturing device 91 returns to the preset shape to clamp the leaflet.

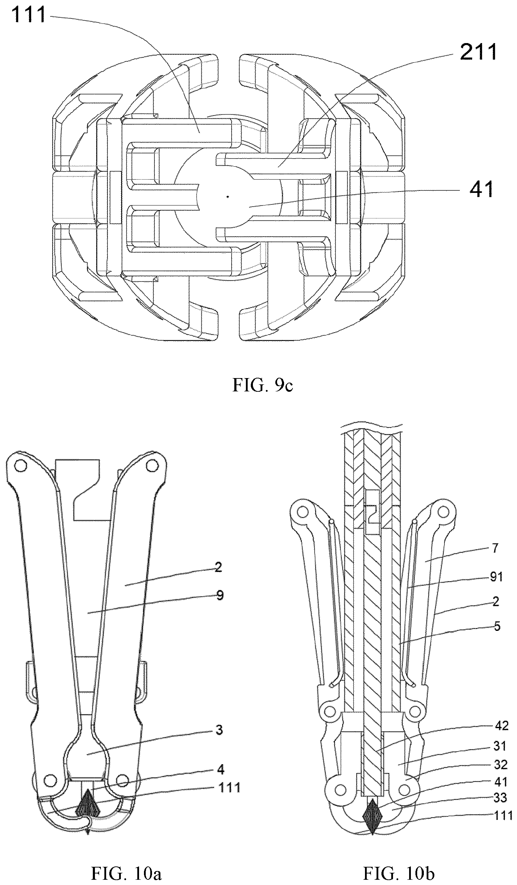

In this example, the locking device 4 is threadedly connected to the connecting member 3. The locking device 4 cannot rotate without external force inside the heart, and thus the locking device 4 cannot move axially when abutting against the first locking part 111 and the second locking part 211. The first locking part 111 and the second locking part 211 are respectively supported by the locking device 4 in a direction opposite to a radial direction of the locking device 4, as shown in FIG. 9c. The first clipping arm 1 is subjected to a radial support force towards the left, and the second clipping arm 2 is subjected to a radial support force towards the right, so that the first clipping arm 1 and the second clipping arm 2 cannot be away from each other. The configurations and connections of various components of the valve delivery system with a locking mechanism of the present application will be described in detail below in conjunction with the accompanying drawings.

In this example, the locking part 111 includes an arc-shaped segment 1111. When the angle formed between the first clipping arm 1 and the second clipping arm 2 becomes large, the arc-shaped segment 1111 of the first clipping arm 1 and the arc-shaped segment 2111 of the second clipping arm 2 are staggered, and fitted into each other. The locking part 111 is designed as the arc-shaped segment 1111 to help increase the force arm of the locking part 111, and enhance the locking force. In addition, the staggered fitting method is beneficial to save the loading space.

In this example, lengths of a first arc-shaped rod 1112 and a second arc-shaped rod 2112 are changed compared with the previous example. When the first clipping arm 1 and the second clipping arm 2 are in a closed state, a hole is formed between the first arc-shaped rod 1112 and the second arc-shaped rod 2112. The locking head 41 of the locking device 4 is arranged in a tapered structure, and a diameter of the proximal end portion of the locking head 41 of the locking device 4 is greater than a distance between an end of the first arc-shaped rod 1112 and an end of the second arc-shaped rod 2112. The locking device 4 is operated so that the distal end portion of the locking head 41 thereof enters/passes through the hole between the first arc-shaped rod 1112 and the second arc-shaped rod 1112. In this case, the locking head 41 also partially abuts against the first arc-shaped rod 1112 and the second arc-shaped rod 1112 to play a role in limiting, so as to realize the locking purpose, as shown in FIGS. 9b and 9c.

### Third specific Example

In another embodiment, as shown in FIGS. 10a and 10b, a valve clip with a locking mechanism includes a first clipping arm 1, a second clipping arm 2, a connecting member 3, and a locking device 4. The locking device 4 is connected to the connecting member 3. The locking device 4 includes a locking head 41 and a self-locking rod 42. The connecting member 3 is hinged to the first clipping arm 1 and the second clipping arm 2 respectively. The clipping arm includes a long arm 12 and a short arm 11. The short arm 11 levers the long arm 12 with a hinge point. An end of the short arm 11 is provided with a locking part 111. When an angle formed between the first clipping arm 1 and the second clipping arm 2 becomes large, the locking part 111 of the first clipping arm 1 and the locking part 111 of the second clipping arm 2 are staggered, and fitted into each other. When the angle formed between the first clipping arm 1 and the second clipping arm 2 becomes small, a locking area 33 is formed between the locking part 111 and the connecting member 3, and an end of the locking device 4 enters the locking area 33 and completes locking cooperation with the locking part 111.

The valve clip further includes a push-pull device 9. The push-pull device 9 includes leaflet capturing devices 91, a leak-proof tube 5, a first linkage rod 6, and a second linkage rod 7, and the first linkage rod 6 and the second linkage rod 7 are hinged to the leak-proof tube 5 and arranged on left and right sides of the leak-proof tube 5. The first linkage rod 6 and the second linkage rod 7 are hinged to the first clipping arm 1 and the second clipping arm 2 respectively. The leaflet capturing devices 91 are arranged on the first linkage rod 6 and the second linkage rod 7. The leaflet capturing device 91 has a preset shape. In the natural state, the leaflet capturing devices 91 are tightly attached to the linkage rods. During pre-installation, the leaflet capturing device 91 is always attached to the leak-proof tube 5. When the valve clip captures the leaflet, the leaflet capturing device 91 returns to the preset shape to clamp the leaflet.

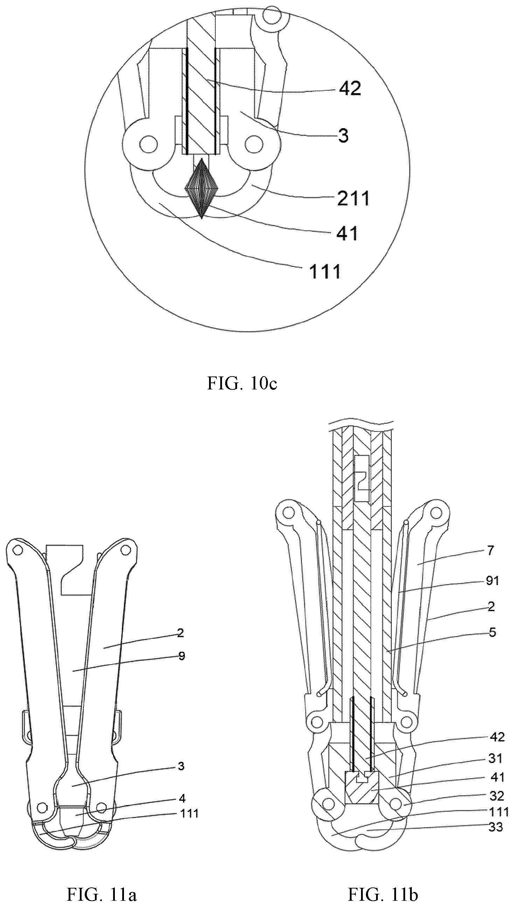

In this example, the self-locking rod 42 is threadedly connected to the connecting member 3. The locking device 4 cannot rotate without external force inside the heart, and thus the locking head 41 cannot move axially when abutting against the first locking part 111 and the second locking part 211. The first locking part 111 and the second locking part 211 are respectively supported by the locking device 4 in a direction opposite to a radial direction of the locking device 4, as shown in FIG. 10c. The first clipping arm 1 is subjected to a radial support force towards the left, and the second clipping arm 2 is subjected to a radial support force towards the right, so that the first clipping arm 1 and the second clipping arm 2 cannot be away from each other. The configurations and connections of various components of the valve delivery system with a locking mechanism of the present application will be described in detail below in conjunction with the accompanying drawings.

Different from the above-mentioned examples, the locking head 41 in this example is a stent-shaped self-expanding structure, and the locking head 41 cooperates with the locking part 111 to realize locking.

In this example, the locking head 41 may be in a tapered structure, a shuttle-shaped structure, a diamond-shaped structure, a prismatic structure, or an arrowhead-shaped structure, and the distal end portion of the locking head 41 is in a tapered structure.

In this example, the locking head 41 is arranged in a diamond-shaped structure, and the locking head 41 is hollow. The locking head 41 is made of an elastic metal material. A hollow area is formed inside the locking head 41, which is a buffering area. When the valve clip is implanted in the heart, the clipping arms will be subject to certain stress due to the compression and relaxation of the heart valve itself, which will affect the clipping degree between the clipping arms and the pushing device, and a certain degree of reflux may occur. However, when the locking head 41 is made of the elastic metal material, and the buffering area is formed inside the locking head 41, the stress received by the long arm 12 of the clipping arm due to the valve's own movement is transmitted to the short arm 11, and then the stress received by the short arm 11 is transmitted to the locking head 41. The locking head 41 is deformed to a certain extent, so that the stress received by the locking head is decomposed, and the clipping strength and tightness between the clipping arm and the push-pull device 9 are always ensured, thereby avoiding reflux.

In this example, the locking part 111 includes a plurality of arc-shaped rods 1112. When the angle formed between the first clipping arm 1 and the second clipping arm 2 becomes large, the arc-shaped rod 1112 of the first clipping arm 1 and the arc-shaped rod 1112 of the second clipping arm 2 are staggered and fitted into each other. The plurality of arc-shaped rods 1112 help to increase the number of action points of the force on the short arm 11, which makes the locking more stable and reliable.

### Fourth specific Example

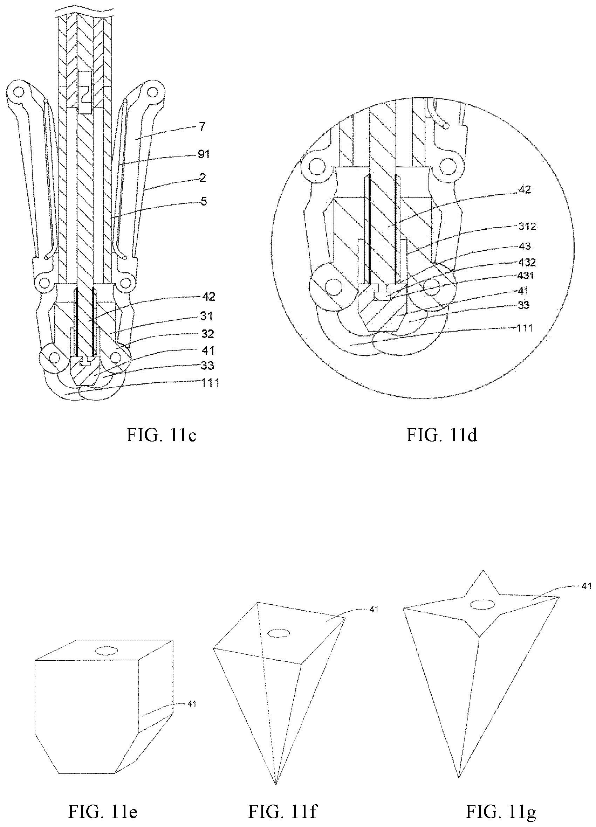

In another embodiment, as shown in FIGS. 11a to 11c, a valve clip with a locking mechanism includes a first clipping arm 1, a second clipping arm 2, a connecting member 3, and a locking device 4. The locking device 4 is connected to the connecting member 3. The locking device 4 includes a locking head 41 and a self-locking rod 42. The connecting member 3 is hinged to the first clipping arm 1 and the second clipping arm 2 respectively. The clipping arm includes a long arm 12 and a short arm 11. The short arm 11 levers the long arm 12 with a hinge point. An end of the short arm 11 is provided with a locking part 111. When an angle formed between the first clipping arm 1 and the second clipping arm 2 becomes large, the locking part 111 of the first clipping arm 1 and the locking part 111 of the second clipping arm 2 are staggered, and fitted into each other. When the angle formed between the first clipping arm 1 and the second clipping arm 2 becomes small, a locking area 33 is formed between the locking part 111 and the connecting member 3, and an end of the locking device 4 enters the locking area 33 and completes locking cooperation with the locking part 111.

The valve clip further includes a push-pull device 9. The push-pull device 9 includes leaflet capturing devices 91, a leak-proof tube 5, a first linkage rod 6, and a second linkage rod 7, and the first linkage rod 6 and the second linkage rod 7 are hinged to the leak-proof tube 5 and arranged on left and right sides of the leak-proof tube 5. The first linkage rod 6 and the second linkage rod 7 are hinged to the first clipping arm 1 and the second clipping arm 2 respectively. The leaflet capturing devices 91 are arranged on the first linkage rod 6 and the second linkage rod 7. The leaflet capturing device 91 has a preset shape. In the natural state, the leaflet capturing devices 91 are tightly attached to the linkage rods. During pre-installation, the leaflet capturing device 91 is always attached to the leak-proof tube 5. When the valve clip captures the leaflet, the leaflet capturing device 91 returns to the preset shape to clamp the leaflet.

In this example, a rotating structure 43 is arranged between the self-locking rod 42 and the locking head 41, so that the self-locking rod 42 and the locking head 41 can rotate relative to each other, as shown in FIG. 11d.

In this example, the rotating structure incudes a groove 431 and a protruding portion 432. The groove 431 is arranged at the proximal end of the locking head 41, and the protruding portion 432 is arranged at the distal end of the self-locking rod 42. The groove 431 cooperates with the protruding portion 432, as shown in FIG. 11d.

In this example, the connecting member 3 is provided with a mounting groove 312. At least part of the locking head 41 is always arranged in the mounting groove 312, and the mounting groove 312 restricts the circumferential rotation of the locking head.

In this example, a shape of the mounting groove 312 matches with a shape of the locking head 41, and the locking head 41 is in a tapered structure, a wedge-shaped structure, a shuttle-shaped structure, or an arrowhead-shaped structure, as shown in FIGS. 11e-11g.

In this example, the connecting member 3 includes a connecting block 31 and connecting lugs 32 arranged on the connecting block 31. The connecting block 31 is connected to the locking device 4. The connecting lugs 32 are hinged connected to the first clipping arm 1 and the second clipping arm 2 respectively. The connecting block 31 is provided with a through hole 311 and the mounting groove 312 in the axial direction. The mounting groove 312 is arranged on the distal side of the through hole 311. When the mounting groove 312 is arranged on the connecting member 3, the weight of the valve clip can be reduced, which can effectively prevent the valve clip from slipping off in the heart due to excessive weight, and is beneficial to the stability of the valve clip anchored in the heart.

In this example, the connecting lugs 32 are symmetrical about a central axis of the connecting block 31.

In this example, the connecting block 31 is provided with the mounting groove 312, at least part of the locking head 41 is always arranged in the mounting groove 312, and the mounting groove 312 restricts the circumferential rotation of the locking head 41. The locking head 41 is completely positioned in the mounting groove 312 when pre-installed, so the loading space can be saved. When the locking head 41 completes locking cooperation with the locking part 111, the locking head 41 is still partly in the mounting groove 312. The purpose of this design is that: when the locking head 41 is arranged in a special-shaped structure, the self-locking rod 42 is operated such that during the locking head 41 axially moving to cooperate with and lock the locking part 111, and the mounting groove 312 restricts the circumferential rotation of the locking head 41, so that the locking head 41 enters the locking area 33 according to a predetermined position and completes cooperation with the locking part.

In this example, the mounting groove 312 is arranged on the distal side of the through hole 311.

In this example, the movement of the locking head 41 towards the proximal end is restricted by the mounting groove 312, and a diameter of the mounting groove 312 is greater than a diameter of the through hole 311.

The above description only illustrates preferred examples of the present application. For those of ordinary skill in the art, according to the idea of the present application, there will be changes in the specific implementation and scope of application, and the contents of this specification should not be construed as a limitation of the present application.

## Claims

1. A valve clip with a locking mechanism, the valve clip comprising a first clipping arm, a second clipping arm, a connecting member, a push-pull device, and a locking device;
wherein the locking device partially cooperates with the connecting member, the locking device comprises a locking head and a self-locking rod, and the connecting member is hinged to the first clipping arm and the second clipping arm respectively;
wherein the clipping arm comprises a long arm and a short arm, an end of the long arm is hinged to the push-pull device, and an end of the short arm is provided with a locking part;
wherein when the push-pull device is operated such that an angle formed between the long arm of the first clipping arm and the long arm of the second clipping arm becomes large and is in an open state, the locking part of the first clipping arm and the locking part of the second clipping arm are partially in a staggered fitting state; and
wherein when the push-pull device is operated such that the angle formed between the long arm of the first clipping arm and the long arm of the second clipping arm becomes small and is in a closed state, the locking device is moved such that the locking head cooperates with and locks the locking part.

2. The valve clip according to claim 1, wherein the short arm is in an arc shape or "L" shape; and
wherein when the angle formed between the first clipping arm and the second clipping arm becomes large, the locking part of the first clipping arm and the locking part of the second clipping arm are staggered, and fitted into each other.

3. The valve clip according to claim 1, wherein the locking head is a stent-shaped self-expanding structure, and the locking head cooperates with the locking part to realize locking.

4. The valve clip according to claim 3, wherein the locking head is in a tapered structure, a shuttle-shaped structure, a diamond-shaped structure, a prismatic structure, or an arrowhead-shaped structure.

5. The valve clip with according to claim 1, wherein a rotating structure is arranged between the self-locking rod and the locking head, so that the self-locking rod and the locking head are capable of rotating relative to each other.

6. The valve clip according to claim 5, wherein the connecting member is provided with a mounting groove, at least part of the locking head is always arranged in the mounting groove, and the mounting groove restricts a circumferential rotation of the locking head.

7. The valve clip according to claim 1, wherein an offset structure is arranged on the long arm; and
wherein the long arm is divided into a fitting portion and a transmission portion by the offset structure, and when the angle formed between the long arm of the first clipping arm and the long arm of the second clipping arm becomes small and is in the closed state, the fitting portion is arranged at a position closer to a center axis of the valve clip than the transmission portion.

8. The valve clip according to claim 1, wherein the connecting member is in an arc shape or in a "V" shape.

9. The valve clip according to claim 1, wherein a cross-section of the long arm is in a concave shape.

10. The valve clip according to claim 1, wherein an end of the long arm connected to the push-pull device is provided with an arc buffering section.
